# EUROPEAN PATENT APPLICATION

(11) **EP 4 570 230 A1**
(43) Date of publication of application: **18.06.2025**
(21) Application number: 23397508.5
(22) Date of filing: 13.12.2023
(51) Int. Cl.: A61K 6/17, A61K 6/30, A61K 6/60, A61K 6/76, C08L 1/02

(54) **A DENTAL BONDING AGENT**

(71) Applicant: Stick Tech OY, 20521 Turku (FI)
(72) Inventor: PELTOLA, Timo, 20740 Turku (FI); SÄILYNOJA, Eija, 20660 Littoinen (FI); VALLITTU, Pekka, 21620 Kuusisto (FI); LASSILA, Lippo, 21630 Lielax (FI); GAROUSHI, Sufyan, 20660 Littoinen (FI)
(74) Representative: Laine IP Oy

(57) **Abstract**

The invention relates to a dental bonding agent comprising a dental adhesive and a calcium phosphate solution. The calcium phosphate solution has 2.5-10 mmol/l of calcium ions and 1.0-4.0 mmol/l of phosphate ions. pH of the calcium phosphate solution is 1-6, provided that the pH of the calcium phosphate solution is such that at least 95 wt-% of the calcium phosphate is in ionic form in the solution. pH of the dental adhesive is at most equal to the pH of the calcium phosphate solution.

## Description

### FIELD OF THE INVENTION

The present invention relates to a dental bonding agent comprising a dental adhesive.

### BACKGROUND AND OBJECTS

Dentin is the major part of a tooth, located under the layer of protective enamel. In the root of the tooth, it is supported by cementum, which attach on the socket with a membrane known as periodontal ligament. Moreover, dentin encloses a space known as pulp cavity, which is filled by pulp. Dentin is generally a vital tissue, and similar to enamel, formed of minerals which concentration is however less compared to the tissue of enamel. Measured by weight, the composition of dentin is 68 % minerals, 21 % organic materials and 11 % of water. The minerals are hydroxyapatite crystals, whereas the organic content of the dentin is 90 % collagen and 10 % non-collagen, including inter alia proteins, lipids, growth factors and enzymes.

Dentin tubules may become demineralised due to for example a diet that contains too much acidic foods or drinks, or following an illness, that reduces the production of saliva. It may thus be necessary to either re-mineralise the dentin or to prevent demineralisation thereof.

Different methods and products for this purpose have been proposed in the art. Document WO 99/34772 presents a solid product for re-mineralising dentin, the solid product comprising a phosphate salt and a calcium salt mixed in a carrier component. The product may be a dental adhesive.

Problems with existing re-mineralising products are that they are not always practical to use or they are not efficient enough. Ideally, the re-mineralising product should possible to incorporate into a product that is in any case used by a dentist, for example in connection with application of a dental restoration. Such a product would be easy to use as it would not require any further steps.

The re-mineralising product should preferably also be such that in long term, in conditions simulating body fluids (i.e. in conditions where the product will be used), the mechanical and other properties of the product will be at least similar and preferably improved compared to known products.

It is thus an aim of the present invention to provide a dental bonding agent that achieves both a good bonding strength and re-mineralises dentin.

### SUMMARY OF THE INVENTION

The invention is described in the enclosed independent claim, the dependent claims defining embodiments of the invention.

The present invention thus relates to a dental bonding agent comprising a dental adhesive and a calcium phosphate solution, wherein
- amount of calcium ions in the calcium phosphate solution is 2.5-10 mmol/l,
- amount of phosphate ions in the calcium phosphate solution is 1.0-4.0 mmol/l,
- pH of the calcium phosphate solution is 1-6, provided that the pH of the calcium phosphate solution is such that at least 95 wt-% of the calcium phosphate is in ionic form in the solution, and
- pH of the dental adhesive is at most equal to the pH of the calcium phosphate solution.

### BRIEF DESCRIPTION OF THE DRAWINGS

Figures 1A and 1B illustrate experimental sample preparation.
Figures 2A and 2B illustrate test setting for measuring shear-bond strength.
Figure 3 illustrates results of shear bond strength.
Figures 4-6 are SEM photos following immersion of samples in SBF at various points of time, along a line cut in the sample.

### DETAILED DESCRIPTION

The present invention relates to a dental bonding agent comprising a dental adhesive and a calcium phosphate solution, wherein
- amount of calcium ions in the calcium phosphate solution is 2.5-10 mmol/l,
- amount of phosphate ions in the calcium phosphate solution is 1.0-4.0 mmol/l,
- pH of the calcium phosphate solution is 1-6, provided that the pH of the calcium phosphate solution is such that at least 95 wt-% of the calcium phosphate is in ionic form in the solution, and
- pH of the dental adhesive is at most equal to the pH of the calcium phosphate solution.

The amounts of calcium ions and phosphate ions in the calcium phosphate solution is in the total volume of the calcium phosphate solution.

In the present invention, a solution that is doped with ions is thus mixed with a dental adhesive, resulting in a dental bonding agent that has improved properties compared to the dental adhesive alone, including re-mineralisation of dentin and in some embodiments improved bonding strength, as shown below in the Experimental part.

Indeed, the present dental bonding agent (later bonding agent) comprises free calcium (Ca²⁺) and phosphate (PO₄³⁻) ions. This means that the bonding agent is able to mineralise dentin, which improves the sealing between the bonding agent and dentin, which in turn is believed to improve bonding strength. Further, it is in general often advantageous to re-mineralise dentin. An additional advantage of the present bonding agent is that the bonding and re-mineralisation are achieved with a single product, that can be applied in one step instead of two or more steps. Furthermore, as is shown below in the Experimental part, the present bonding agent achieves a firm bonding between the dentin and the material applied after the application of the bonding agent (such as a composite material comprising a polymer or a mixture of polymers). The adhesive can be a monomer or a glass ionomer-based material.

The dental adhesive may comprise for example methacryloyloxydecyl dihydrogen phosphate (MDP), which comprises a phosphate group, capable of reacting with calcium. The bonding agent comprises the dental adhesive and the calcium phosphate solution in such amounts that adhesion is achieved, i.e. the bonding agent is a mixture with typically at least 50 vol-% of the adhesive, of the total volume of the bonding agent. According to an embodiment, the calcium phosphate solution is present in an amount of 1-15 vol-% of the total volume of the dental bonding agent. The amount of calcium phosphate solution can thus be for example from 1, 2, 3, 4, 5, 6, 7, 8, 9, 10, 11, or 12 vol-% up to 2, 3, 4, 5, 6, 7, 8, 9, 10, 11, 12, 13, 14, or 15 vol.%, of the total volume of the dental bonding agent. The amount can also be higher, such as up to 20, 25, 30 or 35 vol-%, but usually below 50 vol-% of the total volume.

In the calcium phosphate solution, the amount of calcium ions is 2.5-10 mmol/l and the amount of phosphate ions is 1.0-4.0 mmol/l.

According to an embodiment, the amount of calcium ions is 5-10 mmol/l in the calcium phosphate solution. The amount of calcium ions can thus be for example from 2.5, 3, 3.5, 4, 4.5, 5, 5.5, 6, 6.5, 7, 7.5, 8, 8.5, or 9 mmol/l up to 3, 3.5, 4, 4.5, 5, 5.5, 6, 6.5, 7, 7.5, 8, 8.5, 9, 9.5, or 10 mmol/l. According to another embodiment, the amount of phosphate ions is 2-4 mmol/l. The amount of phosphate ions can thus be for example from 1.0, 1.5, 2, 2.5, 3, or 3.5 mmol/l up to 1.5, 2, 2.5, 3, 3.5, or 4 mmol/l.

Without wishing to be bound by a theory, it is believed that part of the calcium and phosphate ions may react with the dental adhesive. In any case, the calcium and phosphate ions should, at least for the most part, remain free to act and react with the dentin in order to achieve the improved bonding strength and re-mineralisation of dentin. Thus, the pH of the calcium phosphate solution is 1-6, but provided that the pH of the calcium phosphate solution is such that at least 95 wt-% of the calcium phosphate is in ionic form in the solution. It may even be possible to reach higher amounts of calcium phosphate in ionic form, such as up to 97, 98 or even 99 wt-%. This ensures that the ions are readily available to act. This means that for certain concentrations of the ions, the pH needs to be lower than for some other concentrations and depends also on the relative amounts of calcium and phosphate ions. Preferably, the relative amounts of calcium and phosphate are similar or close to that of human body fluids, i.e. the stoichiometric amounts or close to them, needed for the formation of hydroxy apatite. Typically, the lower the pH, the more of the calcium phosphate is in ionic form in the solution.

The pH of the calcium phosphate solution can be for example from 1, 1.5, 2, 2.5, 3, 3.5, 4, 4.5, or 5 up to 1.5, 2, 2.5, 3, 3.5, 4, 4.5, 5, 5.5 or 6. The amount of calcium phosphate in ionic form in the solution can be determined for example by filtering precipitated calcium phosphate out from the solution, weighting it and then calculating the amount of calcium phosphate in ionic form remaining in the solution. This operation only needs to be carried out once when preparing the calcium phosphate solution, when the amounts of calcium and phosphate used are known.

The pH of the dental adhesive is at most equal to the pH of the calcium phosphate solution, i.e. 1-6. According to an embodiment, the pH of the dental adhesive is 1-2. The pH of the dental adhesive can thus be lower than the pH of the calcium phosphate solution. The pH of the bonding agent is thus also 1-6.

According to an embodiment, the dental bonding agent further comprises at least one of cellulose nanocrystals and cellulose nanofibrils.

Cellulose is a biopolymer, which can be found from several different sources, varying from plants to bacteria and algae. Due to the sustainable origin, as well as the high strength and stiffness coupled with relatively low density, cellulose has been widely examined during the past decades. Especially nanocelluloses, which can be further divided to cellulose nanofibrils or nanofibrillar celluloses (NFCs) and cellulose nanocrystals (CNCs), have shown potential to be used as a reinforcement material and modification platform in different biomedical applications. Generally, nanometre scale, isolated cellulose structures are defined as nanocelluloses. Several designations for CNCs can be found in the literature, including whiskers, nanocrystals, nanoparticles, nanofibers, microcrystallites and microcrystals.

By cellulose nanocrystals (CNC) in this context, it is meant a highly crystalline and rigid nanoparticles which are shorter (from some 100 to 1000 nanometres). By cellulose nanofibrils in this context, it is meant a material composed of nanosized cellulose fibrils with a high aspect ratio (length to width ratio). Typical fibril widths are 5-20 nanometres with a wide range of lengths, typically several micrometres.

The length of the fibrils and the size of the crystals can be determined for example by Atomic Force Microscopy (AFM) technique. One possible way of using AFM technique is described in V Lindqvist, "Preparation and characterization of a nanocellulose reinforced light-polymerised methacrylate based dental adhesive", Master's Thesis, Aalto University, 2019, p. 28, which was used in the below Experimental part. The imaging can thus be performed for example with AFM MultiMode 8 scanning probe microscope from Bruker AXS Inc. (Madison, WI, USA) with a J scanner in intermitted contact mode in air. Samples for AFM imaging can be prepared by spin-coating diluted concentrations (100 mg/l) of CNC solutions on silicon wafers that have previously been coated by aqueous 0.1 M solution of titanium (IV) bis(ammonium lactato)dihydroxide (TiO₂-coated wafers), followed by calcination at 600 °C in oven. The AFM images can then be flattened in NanoScope Analysis 1.5 and further used for the particle size determination. Ten images of five different films, each allowing individual nanocrystals to be detected, can then be analysed by image analysis, such as ImageJ. For example, it is possible to measure the largest diameter of all or a number of crystals or width of all or a number of fibrils in each film and calculate the number average (arithmetic mean) of the measurements. Other suitable methods exist and as long as the results are within the above ranges, the present dental bonding agent is believed not to be sensitive to some minor variations that may result from the different measurement methods.

If cellulose nanocrystals or cellulose nanofibrils or both are used, their amount is for example 0.0000033 - 0.0033 wt-% of the total weight of the dental bonding agent. The amount of CNC or NFC is thus very small. Typically, the CNC and/or NFC is in the form of a cellulose-containing solution, which is mixed with the calcium phosphate solution. For example, the cellulose-containing solution can comprise 0.01 - 10 wt-% of the cellulose product.

Again, if cellulose nanocrystals and/or cellulose nanofibrils are used, they can be first mixed with the dental adhesive, before mixing with the calcium phosphate solution, or all solutions or sols can be mixed together at the same time. Cellulose nanocrystals and cellulose nanofibrils are hydrophilic and thus form a reserve of water in the bonding agent. The surface of the dentin is then able to use this stored water from the cellulose, in the re-mineralisation process.

According to an embodiment, the calcium ions and phosphate ions are encapsulated in silica microparticles or in a cellulose nanocrystal sol or hydrogel, i.e. the calcium phosphate solution is encapsulated in silica microparticles or "trapped" in a sol.

Encapsulation as such is known to a person skilled in the art and in general means that the solution comprising the calcium and phosphate ions is fully coated with the encapsulating material. In brief, encapsulation in silica microparticles can be carried out by first making a silicon dioxide-based sol (a colloidal solution), mixing the calcium phosphate solution therein and allowing the gel to form, thereby forming microparticles. The outer surface of the microparticles is formed of porous silicon dioxide (with pores in the nanometer scale) with the calcium phosphate solution therein. The calcium and phosphate ions slowly release from the microcapsules, whereafter the microcapsules also dissolve. Thus, in the beginning, only the outer surface of the microcapsules is in contact with the dental adhesive.

In case of cellulose nanocrystal sol, the CNC and an optional component, such as gelatine or tetraethyl orthosilicate (TEOS), forms a sol or hydrogel in which the calcium phosphate solution is in the liquid phase, as ions have a tendency to migrate to an aqueous environment. The sol may also comprise further components. The sol, when in use mixed with the dental adhesive, then releases the ions to its surrounding tissue. The sol thus forms a sort of carrier for the calcium phosphate solution, the sol forming a continuous colloidal network inside which the calcium phosphate solution is trapped. The sol is most preferably an aqueous sol. A sol is a colloidal suspension of solid particles in a liquid, while a gel is a non-fluid colloidal network or polymer network that is expanded throughout its whole volume by a fluid. A difference between a sol and a gel is for example that same components in low concentration in water for example is a sol, while when higher concentrations are used, the material can be in the form of a gel (hydrogel in case of water).

The calcium phosphate solution can be prepared by diluting K₂HPO₄-3H₂O and CaCl₂-2H₂O in deionised water, and adjusting pH using for example 1 M HCl solution. The pH adjustment results in a clear solution where all material has dissolved. Other sources of ionic phosphate and calcium can also be used.

The dental bonding agent can be prepared by mixing the adhesive and the calcium phosphate solution, for example with a vortex mixer.

In case cellulose nanocrystals and/or cellulose nanofibrils are added to the dental bonding agent, the addition can be done either to the calcium phosphate solution separately, or it may be mixed with the calcium phosphate solution and the adhesive at the same time.

Should the calcium ions and phosphate ions be used encapsulated in silica microparticles, this encapsulation is carried out by mixing the calcium phosphate solution with a source of silica microparticles (such as a solution prepared from tetraethyl orthosilicate). In case the encapsulation is in a cellulose nanocrystal sol, the calcium phosphate solution is first mixed with the cellulose nanocrystals, which CNC may have been surface modified so as to be able to form a sol, followed by mixing with the adhesive. Such surface modification is known as such, and can be for example addition of -COOH or -NH₂ groups, or a sulfate half-ester group.

All mixing steps can be carried out by a suitable mixer, such as a vortex mixer.

### EXPERIMENTAL PART

The present invention was tested on tooth models prepared as explained below.

The following products were used:
- G-aenial Anterior^{™} by GC, a dental composite material
- G-Premio BOND^{™} by GC, a light cured adhesive
- Ncarboxylated nanocrystalline cellulose (CNC) 1 wt-% solution in water, a CNC manufactured by Aalto University, Espoo, see below for more details
- CaCl₂-2 H₂O (manufacturer Sigma Life Science, CAS number: 10035-04-8)
- K₂HPO₄-3 H₂O (manufacturer Sigma-Aldrich, CAS number: 16788-57-1)
- Tetraethyl orthosilicate (TEOS, manufacturer Sigma-Aldrich, CAS number: 78-10-4)

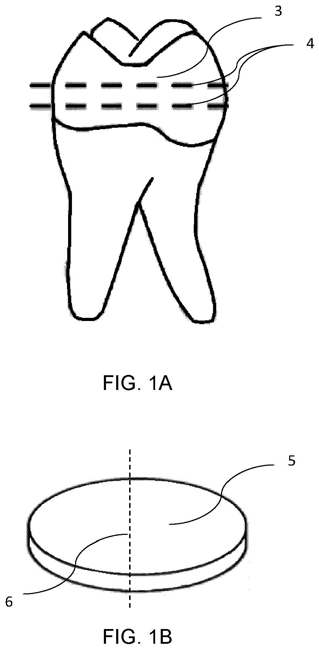

The dental discs used in the testing were prepared as follows. Firstly, the occlusal surfaces of extracted teeth were wet ground using an automatic grinding machine, 500-grit, 300 rpm under water cooling (Struers Rotopol-11) from extracted sound wisdom teeth (acquired from the teaching clinic of Institute of Dentistry, University of Turku, Finland). Thereafter, discs (having a thickness of 2 mm) were cut from the teeth in cross direction of the longest dimension of a tooth, 5 parallel samples for each experiment, as shown in Figure 1A, which illustrates a tooth 3 and two cutting lines 4. Demineralisation of the dentin discs was simulated by acid etching (37 % phosphoric acid for 20 seconds).

The CNC used in the experiments was carboxylated by a process comprising obtaining CNC from beer residuals using alkali extraction, followed by washing. Thereafter the material underwent an acid hydrolysis in pressurised hydrochloride acid gas, in the presence of 2 wt-% NaClO₂ (reaction time 0.67 h), followed by washing and TEMPO oxidation. The resulting product was centrifuged (using Thermo Scientific SL 40 FR, 4500 rpm, 70 minutes, three times) and thereafter dispersed and homogenised using consequently an Ultra Turrax^{™} (pH 9, for 5 minutes in a 1-2 wt-% solution) and Microfluidics M 110P fluidiser (one pass, 100 µm + 200 µm chambers or 200 µm + 400 µm chambers), and finally filtered (Whatman 541). The yield was over 95 %.

A solution of calcium phosphate was prepared by diluting 0.4672 g of K₂HPO₄-3H₂O and 0.7334 g of CaCl₂-2H₂O in 500 ml of deionised water. pH was adjusted to approximatively 3.8 (should be between 3.4-4.7), using 1 M HCl solution. Thereafter the solution became clear and all material was soluble in the solution.

A citrate-phosphate buffer (0.15 M, pH 8.0) / 100 ml was prepared by diluting 1.815 g of Na₂HPO₄-2H₂O (mw: 177.99 g/mol) and 0.961 g of citric acid (mw: 192.1 g/mol) in 80 ml of deionised water. This lead to a pH of 5.0. pH was adjusted to 8.0 with 10 M NaOH solution, poured into 100 ml measuring bottle, where after the bottle was filled to the mark with deionised water until volume was 100 ml.

A 0.032 M tetrahydroxysilane solution was prepared by first mixing 0.025 ml of 1 M HCl and 24.8 ml of deionised water to obtain 25 ml of 1 mM HCl solution. Thereafter, mixing for at least 5 minutes (until the solution is clear) 15.5 ml of the obtained 1 mM HCl solution, 9.2 ml of deionised water, 0.3 ml of TEOS and 0.8 ml of 1 M HCl, to obtain 25 ml of 0.012 M TEOS solution. Further, mixing for at least 5 minutes 22.2 ml of the obtained 0.012 M TEOS solution with 2.8 ml citric acid - phosphate buffer (pH 8.0) obtained as explained above. During mixing, the solution was titrated with 1 M HCl until the solution is clear. Finally, 25 ml of 0.032 M tetrahydroxysilane solution with pH 2.4 was obtained.

The buffer is however not required in the preparation of the tetrahydroxysilane solution, as long as the pH is adjusted to a suitable value, such as 2-4. Indeed, lowering the pH induces reaction between TEOS and water, such that a clear silicon dioxide solution isformed. The solution is opaque or in two phases as long as TEOS has reacted. The desired pH is obtained by titrating with HCl solution.

The dental bonding agents according to the present invention were prepared by mixing the adhesive and the calcium phosphate solution with a vortex mixer. In case the calcium phosphate solution was encapsulated in silica microparticles and CNC was used, the three solutions (calcium phosphate, tetrahydroxysilane, CNC) were mixed in equal volumes (such as 1 ml of calcium phosphate solution, 1 ml of tetrahydroxysilane solution and 1 ml of CNC solution) with a vortex mixture. The desired amount of this mixture was then mixed with the adhesive, using a vortex mixer.

For the tests, the discs (as shown in Figure 1B with reference number 5) were first coated with an adhesive according to the present invention (as listed below in Table 1) and light-cured according to the product instructions. From the SEM photo, the thickness of the adhesive layer was determined to be about 20 µm. Thereafter, a layer of a dental composite material (in the form of a small cylinder having a diameter of 3.7 mm and a height of about 5 mm), G-aenial Anterior^{™} by GC was applied on the adhesive and cured according to the product instructions.

The control sample (C) only had G-Premio BOND^{™} and the dental composite material, applied as above.

Some samples according to the present invention were prepared using varying amounts of calcium phosphate solution, as listed below in Table 1 (Examples 1-6).

**Table 1**

| | **CaP (vol-%)** |
|---|---|
| **Example 1** | 2.5 |
| **Example 2** | 5 |
| **Example 3** | 10 |
| **Example 4** | 12.5 |
| **Example 5** | 15 |
| **Example 6** | 20 |

A sample according to each example as well as the comparative example was stored in water for two days or in simulated body fluid (SBF) prepared according to ISO 23317 (2014) for two days or one month (30 days) and tested for shear bond strength as described below. The only exception were the samples according to Examples 4-6, which were not stored in water.

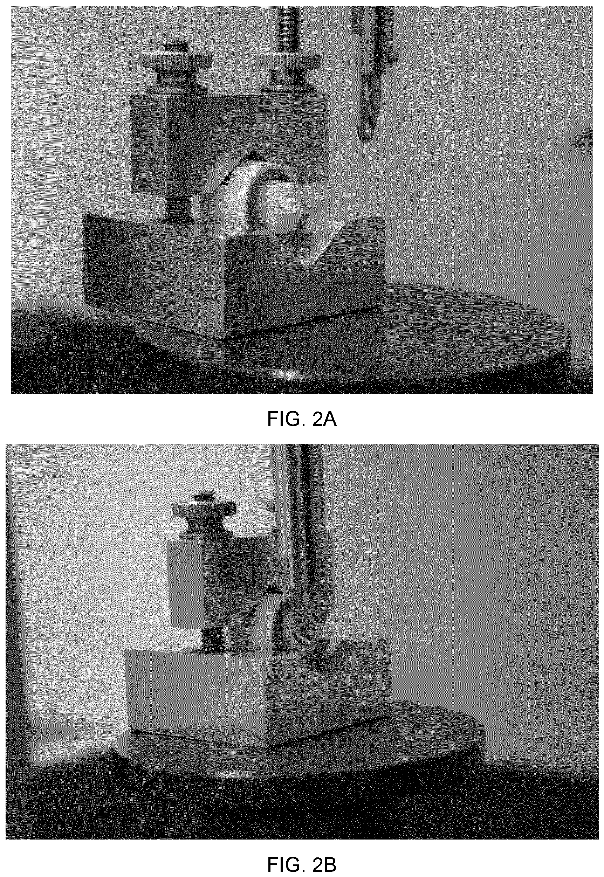

The measurement of bonding performance (shear-bond strength) was carried out as follows. The samples and comparative samples prepared and stored as explained above were first mounted and secured in a mounting jig and then placed on the shear-bond strength test assembly. The test was performed using a universal testing machine (Model LRX, Lloyd Instruments) at room temperature (23 ± 1 °C), and the data was recorded using PC software (Nexygen, Lloyd Instruments). The shearing rod was placed against and parallel to the flat prepared bonding sites. A circular perforation of diameter 4.1 mm was made into the metal blade through which the composites passed through until the metal blade was positioned at the lining material or commercial material-dentin interface, and then using a span length of 10 mm and crosshead speed of 1.0 mm/min, the specimens were loaded until fracture. Bonding strength was calculated by dividing the maximum load at failure (N) with the bonding area (mm2) and recorded in megapascals (MPa). The test setting is illustrated in Figures 2A and 2B.

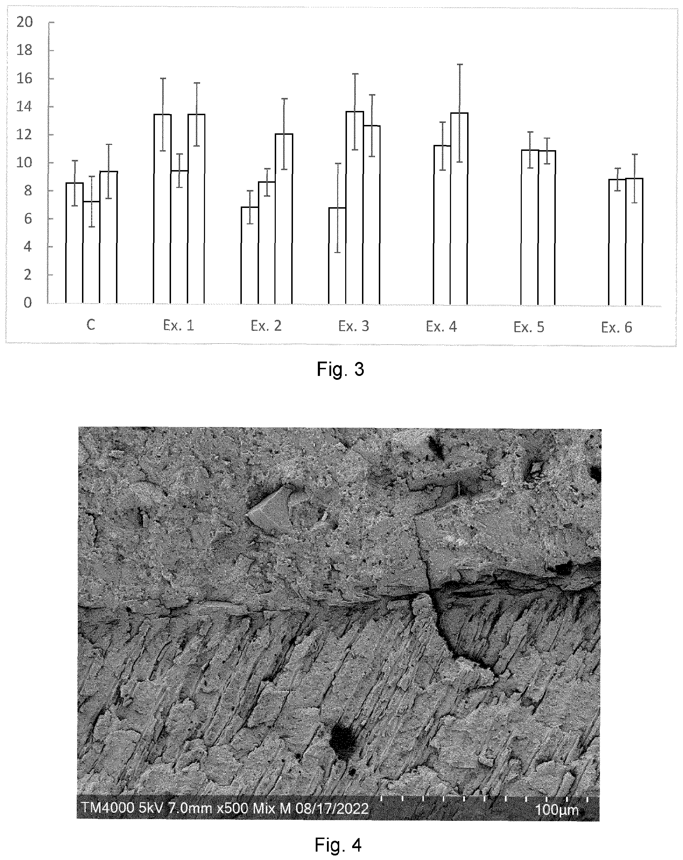

The results of the shear bond strength for Examples (Ex.) 1-6 and the Comparative example (C) are given in Figure 3, the unit being MPa. For the samples C and Ex. 1-3, the left-most column is for storage in water for two days, the middle column for storage in SBF for two days and the right-most column for storage in SBF for 30 days. For the samples Ex. 4-6, the column on the left is for storage in SBF for two days and the column on the right for storage in SBF for 30 days. As can be seen, the results are all better than for the sample according to the Comparative example.

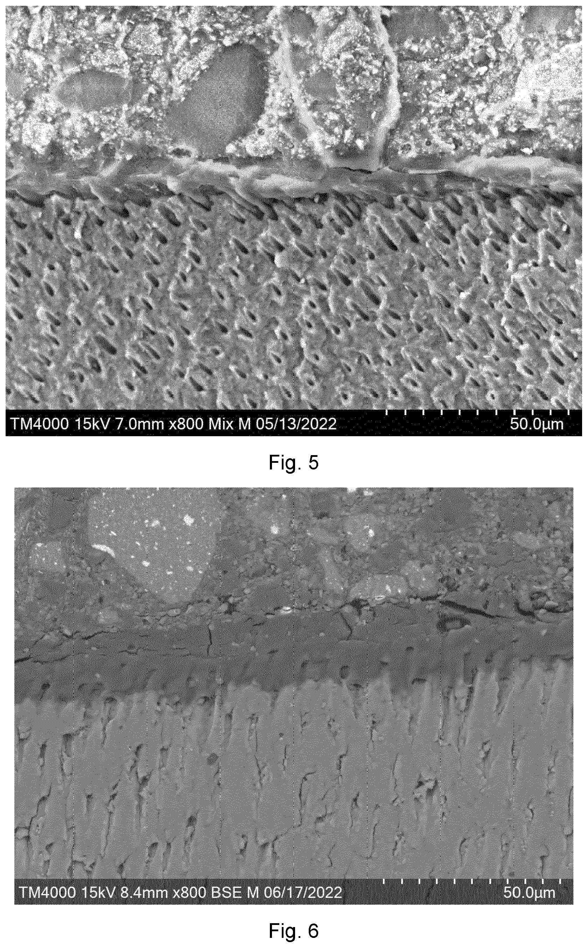

Some samples (after immersion for one month) were also cut along a line (shown with reference number 6 in Figure 1B) and the cutting line was examined under scanning electron microscope (SEM). The results are shown in Figure 4 for Example 1, Figure 5 for Example 4 and Figure 6 for Example 5.

The Figures 4-6 clearly shows firm bonding between adhesive and dentine and composite.

## Claims

1. A dental bonding agent comprising a dental adhesive and a calcium phosphate solution, wherein
- amount of calcium ions in the calcium phosphate solution is 2.5-10 mmol/l,
- amount of phosphate ions in the calcium phosphate solution is 1.0-4.0 mmol/l,
- pH of the calcium phosphate solution is 1-6, provided that the pH of the calcium phosphate solution is such that at least 95 wt-% of the calcium phosphate is in ionic form in the solution, and
- pH of the dental adhesive is at most equal to the pH of the calcium phosphate solution.

2. The dental bonding agent according to claim 1, wherein the calcium phosphate solution is present in an amount of 1-15 vol-% of the total volume of the dental bonding agent.

3. The dental bonding agent according to claim 1 or 2, further comprising at least one of cellulose nanocrystals and cellulose nanofibrils.

4. The dental bonding agent according to claim 3, wherein the amount of cellulose nanocrystals and/or cellulose nanofibrils is 0.0000033 - 0.0033 wt-% of the total weight of the dental adhesive.

5. The dental bonding agent according to any of the preceding claims, wherein the amount of calcium ions is 5-10 mmol/l.

6. The dental bonding agent according to any of the preceding claims, wherein the amount of phosphate ions is 2-4 mmol/l.

7. The dental bonding agent according to any of the preceding claims, wherein the pH of the dental adhesive is 1-2.

8. The dental bonding agent according to any of the preceding claims, wherein the calcium ions and phosphate ions are encapsulated in silica microparticles or in a cellulose nanocrystal sol.
